(19) Europäisches Patentamt
European Patent Office
Office européen des brevets

(11) EP 1 308 153 A2

(12) EUROPÄISCHE PATENTANMELDUNG

(43) Veröffentlichungstag:
**07.05.2003 Patentblatt 2003/19**

(51) Int Cl.[7]: **A61K 7/42**, A61K 31/22, A61K 31/41

(21) Anmeldenummer: **02023341.7**

(22) Anmeldetag: **18.10.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **03.11.2001 DE 10154111**

(71) Anmelder: **Beiersdorf AG**
**20245 Hamburg (DE)**

(72) Erfinder: **Göppel, Anja**
**22527 Hamburg (DE)**

# (54) Insektenabwehrende Sonnenschutzmittel mit Benztriazolderivaten als Lichtschutzfilter

(57) Die vorliegende Erfindung betrifft kosmetische Formulierungen, die mindestens einen UV-Filter mit dem Strukturelement des Benztriazols und mindestens eine Insekten und/oder Spinnen abwehrende Substanz (Repellent) enthalten.

EP 1 308 153 A2

**Beschreibung**

Die vorliegende Erfindung betrifft kosmetische und/oder dermatologische Zubereitungen, die mindestens einen UV-Filter mit dem Strukturelement des Benztriazols und mindestens eine insekten- und/oder spinnenabwehrende Substanz (Repellent) enthalten.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltern entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Zu den in kosmetischen Zubereitungen eingesetzten UV-Lichtschutzfiltern zählt unter anderem Benzophenone, Zimtsäureester, Bornanderivate und Triazine. Auch Benzimidazolsulfonsäuren finden als UV-Lichtschutzfilter Verwendung.

Eine weitere Klasse vorteilhafter Lichtschutzfilter baut auf dem Strukturelement des Benzotriazols auf.

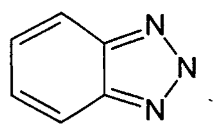

Grundgerüst des Benztriazols

Um die Wirksamkeit der Lichtschutzfilter für die Haut abschätzen zu können, wurde in den 50er Jahren der Lichtschutzfaktor (LSF oder LF) bzw. Sonnenschutzfaktor (SF, engl. sun protection factor SPF) von Schulze eingeführt. Er definiert sich wie folgt:

$$LSF = \frac{\textit{MED geschützte Haut}}{\textit{MED ungeschützte Haut}}$$

*MED* = Erythemschwellendosis (engl. minimum erythemal dose)

Eine Vielzahl von Mücken, Bremsen Flöhen, Läusen, Wanzen, sowie Zecken und Milben, im weiteren Verlauf unter den Sammelbegriffen Insekten und Spinnen zusammengefasst, ernähren sich vom Blut der Warmblüter, zu denen auch die Menschen zählen. Sie bohren sich mit ihren Stech- und Saugwerkzeugen in die Haut ihrer Opfer bis sie auf Blutgefäße stoßen. Während der Nahrungsaufnahme sondern sie gefäßerweiternde und gerinnungshemmende Mittel ab, die beim Wirt zu Juckreiz, Quaddelbildung und allergischen Reaktionen führen können. Insbesondere in den Tropen und Subtropen besteht darüber hinaus die Gefahr einer Infektion mit Krankheitserregern. So wird beispielsweise die Malaria durch die Anopheles-Mücke oder das Gelbfieber durch die Gelbfiebermücke übertragen. Auch in gemäßigten Regionen besteht die Gefahr von Infektionen mit von Insekten übertragenen Krankheitserregern wie beispielsweise der durch Zeckenbiss übertragenen Zeckenenzephalitis.

Einen Schutz vor der Belästigung durch Insekten und Spinnen bieten sogenannte Repellentien. Darunter versteht man eine Reihe von Wirksubstanzen, die durch ihren Geruch abweisend auf Insekten und Spinnen wirken. Dabei handelt es sich in der Regel um schwerflüchtige Verbindungen, die langsam auf der Haut verdampfen und somit eine Duftglocke über der Haut bilden, welche die Insekten vertreibt.

Zu den gebräuchlichsten Repellentien zählt N,N-Diethyl-3-methylbenzamid (Handelsbezeichnung: Meta-delphene, "DEET"), das gegen Stechmücken, Stech- und Sandfliegen, Bremsen, Flöhe, Wanzen, Zecken und Milben aktiv ist.

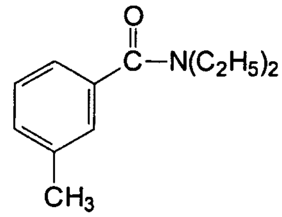

Ferner wird Dimethylphtalat (Handelsbezeichnung: Palatinol M, DMP) gegen Stechmücken, Läuse, Zecken und Milben eingesetzt.

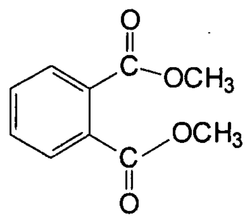

Besonders wirksam ist 3-(N-n-Butyl-N-acetyl-amino)-propionsäureethylester (unter dem Handelsnamen Insekt Repellent® 3535 bei der Fa. Merck erhältlich), dass gegen Stechmücken, Tsetsefliegen und Bremsen eingesetzt werden kann.

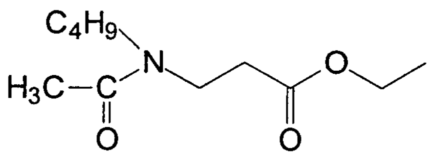

In vielen Regionen der Erde besteht nicht nur am Abend die Gefahr von Insekten gestochen zu werden. Auch tagsüber sollte man sich mit Repellentien schützen. Gleichzeitig ist es tagsüber aber auch außerordentlich sinnvoll, sich mit Hilfe von Sonnenschutzmitteln vor der schädlichen ultravioletten Strahlung des Sonnenlichtes zu schützen. Derzeit muss man in der Regel zum Schutz vor Insekten und Spinnen einerseits und zum Schutz vor UV-Strahlung andererseits unterschiedliche Produkte verwenden. Nur wenige Produkte gewährleisten einen gewissen Schutz sowohl vor der UV-Strahlung als auch vor Angriffen durch Insekten und Spinnen. Es war deshalb die Aufgabe der vorliegenden Erfindung ein insektenabweisendes Sonnenschutzmittel zu entwickeln.

Überraschend gelöst wird die Aufgabe durch kosmetische und/oder dermatologische Zubereitungen, die mindestens einen UV-Filter mit dem Strukturelement des Benztriazols und mindestens eine insekten- und spinnenabweisende Substanz (Repellent) enthalten.

Durch die Kombination von UV-Lichtschutzfiltern auf Basis von Benztriazolderivaten und Repellentien ist es möglich, Zubereitungen zum Schutz vor Insekten und Spinnen zu entwickeln, die sich gleichzeitig durch einen sehr guten Schutz der Haut vor UV-Strahlung auszeichnen, da das Repellent in überraschender Weise den Sonnenschutzfaktor (UVA + UVB) erhöht. Dies war nicht vorhersehbar, da den Repellentien für sich genommen keine vor UV-Strahlung schützende Wirkung nachgesagt wird.

Überraschenderweise wird durch Zusatz von Repellentien auch die Verteilung der organischen, unlöslichen Benztriazolderivate auf der Haut sowie deren Verteilung in den Zubereitungen deutlich verbessert. Für lösliche Benzotriazolderivate wird ebenfalls deren gleichmäßige Verteilung in den Zuberreitungen verbessert und dadurch deren Löslichkeit stark erhöht.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch eine Mikroemulsion, eine Feststoff-Emulsionen (d. h. eine Emulsion, welche durch Feststoffe stabilisiert ist, z. B. eine Pickering-Emulsion), eine sprühbare Emulsion oder eine Hydrodispersion sein. Vorteilhafte Ausführungsformen der erfindungsgemäßen Zubereitungen stellen beispielsweise Salben, Cremes und Lotionen dar. Des Weiteren können die Formulierungen im Sinne

der vorliegenden Erfindung auch wasserfreie Systeme (Puder, Öle oder Wachsstifte) oder ölfreie Systeme (wässrige, wässrig/alkoholisch oder nur alkoholische Lösungen) sein.

Die erfindungsgemäßen Zubereitungen stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsforrrien mit vielfältigen Anwendungszwecken zu dienen. Die erfindungsgemäßen Zubereitungen zeigen sehr gute sensorische und kosmetische Eigenschaften, wie beispielsweise die Verteilbarkeit auf der Haut oder das Einzugsvermögen in die Haut, und zeichen sich ferner durch eine sehr gute Lichtschutzeffektivität (UVA & UVB) bei gleichzeitig hervorragenden Hautpflegedaten aus.

Einige Benzotriazole zeichnen sich durch die folgende Strukturformel aus:

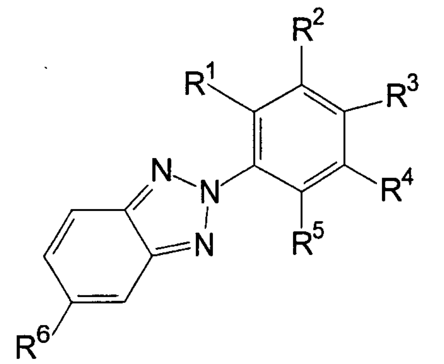

Worin:

$R^1$, $R^2$, $R^3$, $R^4$, $R^5$, $R^6$ unabhängig voneinander lineare oder verzweigte, gesättigte oder ungesättigte, substituierte (z. B. mit einem Phenylrest substituierte) oder unsubstituierte Alkylreste mit 1 bis 18 Kohlenstoffatomen und/oder Polymerreste, welche selbst nicht UV-Strahlen absorbieren (wie z. B. Silikonreste, Acrylatreste und dergleichen mehr), ein Wasserstoff, eine OH-Gruppe oder eine Sulfonsäure-Gruppe darstellen können. Alle diese Benztriazolderivate können erfindungsgemäß vorteilhaft in die erfindungsgemäßen Zubereitungen eingearbeitet werden.

Vorteilhafte Benzotriazole im Sinne der vorliegenden Erfindung sind [2,4'-Dihydroxy-3-(2H-benzotriazol-2-yl)-5-(1,1,3,3-tetramethylbutyl)-2'-n-octoxy-5'-benzoyl]diphenylmethan,2,2'Methylen-bis-[6-(2H-benzotriazol-2-yl)-4-(methyl)phenol], 2,2'-Methylenebis-[6-(2H-benzotiazol-2-yl)-4-(1,1,3,3-trtramethylbutyl)phenol], 2-(2'-Hydroxy-5'octylphenyl)-benzotriazol, 2-(2'-Hydroxy-3',5'-di-t-amylphenyl)benzotriazol und 2-(2'-Hydroxy-5'-methylphenyl)benzotriazol.

Ein besonders vorteilhaftes Benzotriazol im Sinne der vorliegenden Erfindung ist das 2,2'-Methylen-bis-(6-(2H-benzotriazol-2-yl)-4-(1,1,3,3-tetramethylbutyl)-phenol) (Methylen Bis-Benzotriazolyl Tetramethylbutylphenol), ein Breitbandfilter, welcher durch die chemische Strukturformel

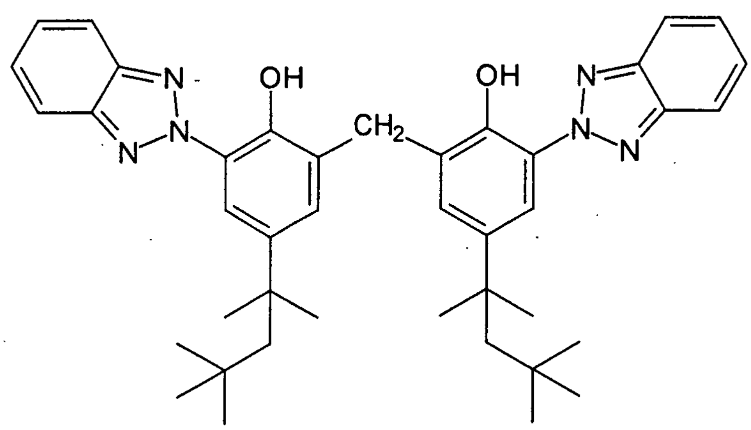

gekennzeichnet ist. Dieser ist beispielsweise unter der Handelsbezeichnung Tinosorb® M bei der CIBA-Chemikalien

GmbH erhältlich ist. 2,2'-Methylen-bis-(6-(2H-benzotriazol-2-yl)-4-(1,1,3,3-tetramethylbutyl)-phenol) kann auch als nicht löslicher (dadurch pigmentärer), organischer Breitbandfilter eingesetzt werden. Die mittlere Partikelgröße liegt erfindungsgemäß vorteilhaft zwischen 0,01 µm und 0,20 µm

Ein weiteres besonders vorteilhaftes Benzotriazol im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCI-Bezeichnung Drometrizole Trisiloxane, welches durch die chemische Strukturformel

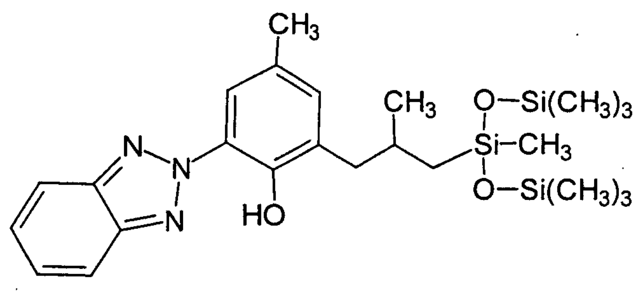

gekennzeichnet ist.

Erfindungsgemäß enthalten kosmetische oder dermatologische Zubereitungen 0,1 bis 20 Gew.-%, vorteilhaft 0,5 bis 15 Gew.-%, ganz besonders bevorzugt 0,5 bis 10 Gew.-% eines oder mehrerer Benzotriazole.

Erfindungsgemäß vorteilhafte Repellentien sind Dimethylphtalat und N,N-Diethyl-3-methylbenzamid.

Als besonders vorteilhaft im Sinne der vorliegenden Erfindung erweist sich 3-(N-n-Butyl-N-acetyl-amino)-propionsäureethylester (Repellent 3535) als Repellent.
Als erfindungsgemäß vorteilhaft ist dabei eine Konzentration an 3-(N-n-Butyl-N-acetylamino)-propionsäureethylester (Repellent 3535) von 1-25 Gewichts-% bezogen auf das Gesamtgewicht der Formulierung.
Ganz besonders vorteilhaft im Sinne der Erfindung ist dabei der Mengenbereich von 4 bis 20 Gewichts-% bezogen auf das Gesamtgewicht der Formulierung.

Daher ist es von Vorteil UV-Filter und Repellentien im Verhältnis von 0,1 : 1 bis zu einem Verhältnis von 1 :1 einzusetzen.

Kosmetische und/oder dermatologische Zubereitungen im Sinne der vorliegenden Erfindung können vorzugsweise mindestens eine weitere UV-A-, UV-B- und/oder Breitbandfiltersubstanz enthalten. Die Zubereitungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfatoverbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;

- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.

- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
- Diethylhexylbutylamidotriazon (INCI: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Die weiteren UV-Filtersubstanzen können öllöslich oder wasserlöslich sein.

Vorteilhafte öllösliche UV-B- und/oder Breitband-Filtersubstanzen im Sinne der vorliegenden Erfindung sind z. B.:

- ■ 3-Benzylidencampher-Derivate, vorzugsweise 3-(4-Methylbenzyliden)campher, 3-Benzylidencampher;
- ■ 4-Aminobenzoesäure-Derivate, vorzugsweise 4-(Dimethylamino)-benzoesäure(2-ethylhexyl)ester, 4-(Dimethylamino)benzoesäureamylester;
- ■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- ■ sowie an Polymere gebundene UV-Filter.
- ■ (3-(4-(2,2-bis Ethoxycarbonylvinyl)-phenoxy)propenyl)-methoxysiioxan/Dimethylsiloxan- Copolymer welches beispielsweise unter der Handelsbezeichnung Parsol® SLX bei Hoffmann La Roche erhältlich ist.

  Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethylhexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul $TiO_2$) | Octyltrimethylsilan | Degussa |

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt. Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Die erfindungsgemäßen kosmetische oder dermatologische Lichtschutzzubereitungen können 0,1 bis 20 Gew.-%, vorteilhaft 0,5 bis 10 Gew.-%, ganz besonders bevorzugt 1 bis 5 Gew.-% röntgenamorphe Oxidpigmente enthalten.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Die kosmetischen und dermatologischen Zubereitungen gemäß der Erfindung können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feucht haltende Substanzen, Fette, Öle, Wachse oder andere übliche Bestandteile einer kosmetischen oder dermatologischen Formulierung wie Alkohole, Polyole, Polymere, Schaumstabilisatoren,

Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), Iodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparabeh), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Auch ein Gehalt an Antioxidantien ist im allgemeinen in den erfindungsgemäßen Zubereitungen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$ -Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05-20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das

Gesamtgewicht der Formulierung, zu wählen.

Die Lipidphase von erfindungsgemäßen Zubereitungen kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.
- Salicylate

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether *(Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Dlglycerylsuccinat, Butylenglykol Dicaprylat/Dicaprat, $C_{12-13}$-Alkyllactat, Di-$C_{12-13}$-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an $C_{12-15}$-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB)* und/oder Diethylhexylnaphthalat (*Hallbrite TQ bzw.Corapan® TQ*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der

Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Oelphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen

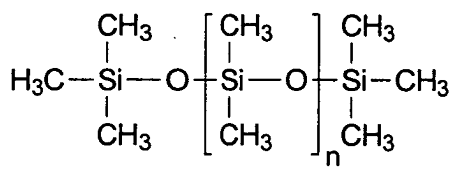

werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäße, als Emulsionen vorliegende Zubereitungen enthalten einen oder mehrere Emulgatoren.

O/W-Emulgatoren können beispielsweise vorteilhaft gewählt. werden aus der Gruppe der polyethoxylierten bzw. polypropoxylierten bzw. polyethoxylierten und polypropoxylierten Produkte, z.B.:

- der Fettalkoholethoxylate
- der ethoxylierten Wollwachsalkohole,
- der Polyethylenglycolether der allgemeinen Formel $R\text{-}O\text{-}(\text{-}CH_2\text{-}CH_2\text{-}O\text{-})_n\text{-}R'$,
- der Fettsäureethoxylate der allgemeinen Formel

$$R\text{-}COO\text{-}(\text{-}CH_2\text{-}CH_2\text{-}O\text{-})_n\text{-}H,$$

- der veretherten Fettsäureethoxylate der allgemeinen Formel

$$R\text{-}COO\text{-}(\text{-}CH_2\text{-}CH_2\text{-}O\text{-})_n\text{-}R',$$

- der veresterten Fettsäureethoxylate der allgemeinen Formel

$$R\text{-}COO\text{-}(\text{-}CH_2\text{-}CH_2\text{-}O\text{-})_n\text{-}C(O)\text{-}R',$$

- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

$R\text{-}O\text{-}(\text{-}CH_2\text{-}CH_2\text{-}O\text{-})_n\text{-}CH_2\text{-}COOH$ nd n eine Zahl von 5 bis 30 darstellen,

- der Polyoxyethylensorbitolfettsäureester,
- der Alkylethersulfate der allgemeinen Formel $R\text{-}O\text{-}(\text{-}CH_2\text{-}CH_2\text{-}O\text{-})_n\text{-}SO_3\text{-}H$
- der Fettalkoholpropoxylate der allgemeinen Formel

$$R\text{-}O\text{-}(\text{-}CH_2\text{-}CH(CH_3)\text{-}O\text{-})_n\text{-}H,$$

- der Polypropylenglycolether der allgemeinen Formel

$$R\text{-}O\text{-}(\text{-}CH_2\text{-}CH(CH_3)\text{-}O\text{-})_n\text{-}R',$$

- der propoxylierten Wollwachsalkohole,
- der veretherten Fettsäurepropoxylate

$$R\text{-}COO\text{-}(\text{-}CH_2\text{-}CH(CH_3)\text{-}O\text{-})_n\text{-}R',$$

- der veresterten Fettsäurepropoxylate der allgemeinen Formel

$$R\text{-}COO\text{-}(\text{-}CH_2\text{-}CH(CH_3)\text{-}O\text{-})_n\text{-}C(O)\text{-}R',$$

- der Fettsäurepropoxylate der allgemeinen Formel

$$R\text{-}COO\text{-}(\text{-}CH_2\text{-}CH(CH_3)\text{-}O\text{-})_n\text{-}H,$$

- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

$$R\text{-}O\text{-}(\text{-}CH_2\text{-}CH(CH_3)O\text{-})_n\text{-}CH_2\text{-}COOH$$

- der Alkylethersulfate bzw. die diesen Sulfaten zugrundeliegenden Säuren der allgemeinen Formel $R\text{-}O\text{-}(\text{-}CH_2\text{-}CH(CH_3)\text{-}O\text{-})_n\text{-}SO_3\text{-}H$
- der Fettalkoholethoxylate/propoxylate der allgemeinen Formel

$$R\text{-}O\text{-}X_n\text{-}Y_m\text{-}H,$$

- der Polypropylenglycolether der allgemeinen Formel

$$R\text{-}O\text{-}X_n\text{-}Y_m\text{-}R',$$

- der veretherten Fettsäurepropoxylate der allgemeinen Formel

$$R\text{-}COO\text{-}X_n\text{-}Y_m\text{-}R',$$

- der Fettsäureethoxylate/propoxylate der allgemeinen Formel

$$R\text{-}COO\text{-}X_n\text{-}Y_m\text{-}H,.$$

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18) stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19-), Polyethylenglycol (20)isostearylether (Isosteareth-20),

Polyethylenglycol(13)cetylether (Ceteth-13), Polyethylenglycol(14)cetylether (Ceteth-14), Polyethylenglycol (15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17) isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol (14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21 )stearat, Polyethylenglycol(22)stearat, Polyethylenglycol(23) stearat, Polyethylenglycol(24)stearat, Polyethylenglycol(25)stearat,

Polyethylenglycol(12)isostearat, Polyethylenglycol(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21 )isostearat, Polyethylenglycol-(22)isostearat, Polyethylenglycol(23)isostearat, Polyethylenglycol(24)isostearat, Polyethylenglycol (25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natriumlaurylethersulfat mit 1 bis 4 Oxyethyleneinheiten vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20) glyceryllaurat, Polyethylenglycol(21 )glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/ oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Liegen die erfindungsgemäßen Zubereitungen als Emulsion mit W/O-Emulgatoren vor, können diese W/ O-Emulgatoren vorteilhaft gewählt werden aus der Gruppe Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß können auch Siliconemulgatoren, und zwar vorteilhaft aus der Gruppe grenzflächenaktive Substanzen aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur:

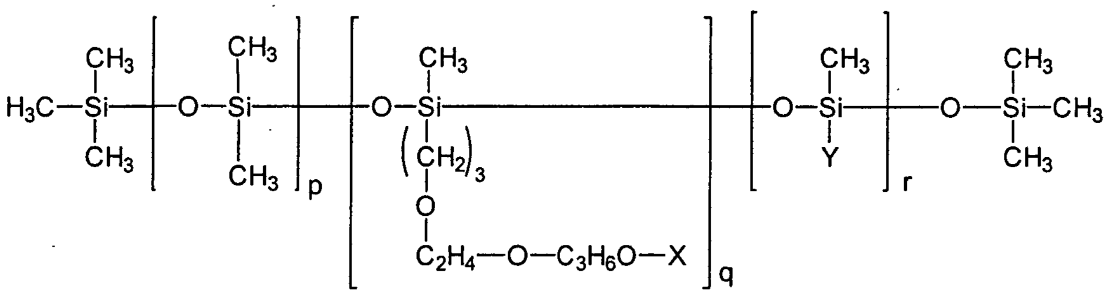

bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Ein Beispiel für besonders vorteilhaft im Sinne der vorliegenden Erfindung zu verwendende Silikonemulgatoren sind Dimethiconcopolyole, welche von der Gesellschaft Th.Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden.

Ein weiteres Beispiel für besonders vorteilhaft im Sinne der vorliegenden Erfindung zu verwendende grenzflächenaktiven Substanzen ist das Cetyl Dimethiconcopolyol, welches von der Gesellschaft Th.Goldschmidt AG unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Th.Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an erfindungsgemäß verwendeten Siliconemulgatoren in den erfindungsgemäßen kosmetischen oder dermatologischen Zubereitungen wird vorteilhaft aus dem Bereich von 0,1 - 10,0 Gew.-%, bevorzugt 0,5 - 5,0 Gew.-% gewählt, bezogen auf das Gesamtgewicht der Zubereitungen.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP)

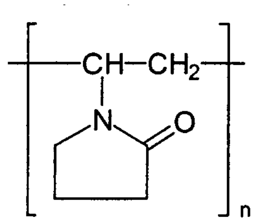

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Insbesondere ist es im Sinne der Erfindung vorteilhaft, die kosmetische Formulierung als Sonnenschutzmittel und/oder Insektenabwehrmittel zu verwenden.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiele:**

| **1. O/W Sonnenschutz Emulsionen** | | | | | | | |
|---|---|---|---|---|---|---|---|
| | **1** | **2** | **3** | **4** | **5** | **6** | **7** |
| Glycerinmonostearat SE | 0,50 | 1,00 | 3,00 | | | 1,50 | |
| Glycerylstearatcitrat | 2,00 | | | 1,00 | | | 2,50 |
| Laurylmethicone Copolyol | | 0,50 | | | | | 1,00 |
| Stearinsäure | | 3,00 | | 2,00 | | | |
| PEG-100 Stearat | 0,50 | | | | 1,00 | 2,00 | |
| Cetylphosphat | | | | | 1,00 | | |
| Stearylalkohol | | | 3,00 | | | 2,00 | 0,50 |
| Cetylalkohol | 2,50 | 1,00 | | 1,50 | | | 2,00 |
| Ethylhexylmethoxycinnamat | | | | 5,00 | 6,00 | | |
| Bis-Ethylhexyloxyphenolmethoxyphenyltriazin | | 1,50 | | 2,00 | | | 2,50 |
| Butylmethoxydibenzoylmethan | | 3,00 | | | 2,80 | 2,00 | |
| Dinatriumphenyldibenzimidazoltetrasulfonat | 2,50 | 0,50 | | 2,00 | | | 0,30 |
| Ethylhexyltriazon | | | | 4,00 | | 2,00 | |
| 4-Methylbenzylidencampher | 4,00 | 4,00 | | | 2,00 | 4,00 | |
| Octocrylen | | 4,00 | | | | | 2,50 |
| Diethylhexylbutamidotriazon | 1,00 | | | 2,00 | 1,00 | | |
| Phenylbenzmidazolsulfonsäure | 0,50 | | | 3,00 | | | |
| Methylen Bis-Benzotriazolyl Tetramethylbutylphenol | 2,00 | | | 1,50 | 2,50 | | 6,00 |
| Drometrizol Trisiloxan | | 2,00 | 1,00 | 5,00 | | 1,00 | 3,00 |
| Terephthalidendicamphersulfon säure | | 1,50 | | | 1,00 | 0,50 | |
| Repellent 3535 | 5,0 | 10,00 | 25 | 15,5 | | | 18 |
| N,N-Diethyl-3-methylbenzamid | | | | | 20 | 12 | |
| Titandioxid MT-100Z | 1,00 | 1,50 | | 3,00 | 2,00 | 2,00 | |
| Zinkoxid HP1 | | | | 1,00 | | 2,00 | 3,00 |
| C12-15 Alkylbenzoat | | 2,50 | | | 4,00 | 7,00 | 5,00 |
| Dicaprylylether | | | 3,50 | | 2,00 | | |
| Butylenglycol Dicaprylat/Dicaprat | 5,00 | | | 6,00 | | | |
| Dicaprylylcarbonat | | | 6,00 | | | 2,00 | 2,00 |
| Dimethicon | | 0,50 | 1,00 | | 2,00 | | |
| Cyclomethicon | 2,00 | | | 0,50 | | | 0,50 |
| Shea Butter | | 2,00 | | | | | 0,50 |

(fortgesetzt)

| 1. O/W Sonnenschutz Emulsionen | | | | | | | |
|---|---|---|---|---|---|---|---|
| | **1** | **2** | **3** | **4** | **5** | **6** | **7** |
| PVP Hexadecen Copolymer | 0,50 | | | 0,50 | 1,00 | | 1,00 |
| Tricontanyl PVP | | 0,50 | 1,00 | | | | 1,00 |
| Glycerin | 3,00 | 7,50 | | 7,50 | 5,00 | | 2,50 |
| Xanthan Gummi | 0,15 | | 0,05 | | | | 0,30 |
| Sodium Carbomer | | 0,20 | 0,10 | 0,20 | | | |
| Vitamin E Acetat | 0,50 | | 0,25 | | 0,75 | | 1,00 |
| EDTA | | 0,05 | | 0,03 | | | |
| DMDM Hydantoin | | 0,60 | 0,40 | 0,20 | | | |
| Konkaben LMB ® | | | | 0,18 | 0,20 | | 0,15 |
| Methylparaben | 0,15 | | 0,25 | | 0,50 | | |
| Phenoxyethanol | 1,00 | 0,40 | | 0,40 | | | 0,60 |
| Ethanol | | 2,00 | 1,50 | | 3,00 | 5,00 | 1,00 |
| Parfüm | 0,20 | 0,20 | | 0,20 | | 0,20 | 0,20 |
| Wasser | ad 100 | ad 100 | ad 100 | ad 100 | ad 100 | ad 100 | ad 100 |

| 2. Hydrodispersionen | | | | | |
|---|---|---|---|---|---|
| | **1** | **2** | **3** | **4** | **5** |
| Ceteareth-20 | 1,00 | | | 0,5 | |
| Cetylalkohol | | | 1,00 | | |
| Natrium Carbomer | | 0,20 | | 0,30 | |
| Acrylates/C10-30 Alkyl Acrylat Crosspolymer | 0,50 | | 0,40 | 0,10 | 0,10 |
| Xanthangummi | | 0,30 | 0,15 | | 0,50 |
| Ethylhexylmethoxycinnamat | | | | | 8,00 |
| Bis-Ethylhexyloxyphenolmethoxyphenyltriazin | | 1,50 | | | 2,50 |
| Butylmethoxydibenzoylmethan | 1,00 | | | | 2,50 |
| Dinatriumphenyldibenzimidazoltet rasulfonat | 0,50 | 1.80 | 1,50 | | 3.00 |
| Ethylhexyltriazon | 4,00 | | 3,00 | | |
| Octocrylen | | 4,00 | 3,90 | | 2,50 |
| Diethyhexylbutamidotriazon | 1,00 | | | | |
| Phenylbenzmidazolsulfonsäure | 0,50 | | | | |
| Methylen Bis-Benzotriazolyl Tetramethylbuthylphenol | 2,50 | 1,50 | | 6,00 | 0,80 |
| Drometrizol Trisiloxan | | | 1,00 | 2,00 | 1,50 |
| Terephthalidendicamphersulfonsäure | | 0,50 | | | 1,00 |
| Repellent 3535 | 5,0 | 12,0 | 5,0 | 22,5 | 18 |
| Titandioxid MT-100TV | 0,50 | | 2,00 | | 1,00 |

(fortgesetzt)

| 2. Hydrodispersionen | | | | | |
|---|---|---|---|---|---|
| | **1** | **2** | **3** | **4** | **5** |
| C12-15 Alkylbenzoat | 2,00 | 2,50 | | | |
| Dicaprylylether | | 4,00 | | | |
| Butylenglycol Dicaprylat/Dicaprat | 4,00 | | 2,00 | 6,00 | |
| Dicaprylylcarbonat | | 2,00 | 6,00 | | |
| Dimethicon | | 0,50 | 1,00 | | |
| Phenyltrimethicon | 2,00 | | | 0,50 | 2,00 |
| Shea Butter | | 2,00 | | | |
| Tricontanyl PVP | 0,50 | | 1,00 | | |
| Ethylhexylglycerin | | | 1,00 | | 0,50 |
| Glycerin | 3,00 | 7,50 | | 7,50 | 2,50 |
| Glycin Soja | | | 1,50 | | |
| Vitamin E Acetat | 0,50 | | 0,25 | | 1,00 |
| DMDM Hydantoin | | 0,60 | 0,40 | 0,20 | |
| Konkaben LMB ® | 0,20 | | | | 0,15 |
| EDTA | 0,10 | | | 0,05 | |
| Methylparaben | 0,50 | | 0,25 | 0,15 | |
| Phenoxyethanol | 0,50 | 0,40 | | 1,00 | 0,60 |
| Ethanol | 3,00 | 2,00 | 1,50 | | 1,00 |
| Parfüm | 0,20 | | | 0,20 | 0,20 |
| Wasser | ad 100 | ad 100 | ad 100 | ad 100 | ad 100 |

| 3. W/O Sonnenschutz Emulsionen | | | | | |
|---|---|---|---|---|---|
| | **1** | **2** | **3** | **4** | **5** |
| Cetyldimethicon Copolyol | | 2,50 | 2,00 | 4,00 | |
| Polyglyceryl-2-dipolyhydroxystearat | 5,00 | | | | 4,50 |
| PEG-30-dipolyhydroxystearat | | | 5,00 | | |
| Lauryldimethicon Copolyol | 2,00 | | | 1,00 | |
| Ethylhexylmethoxycinnamat | | 8,00 | | 5,00 | 4,00 |
| Bis-Ethylhexyloxyphenolmethoxyphenyltriazin | 2,00 | 2,50 | | 2,00 | 2,50 |
| Butylmethoxydibenzoylmethan | 0.50 | 3,00 | | | 0,70 |
| Dinatriumphenyldibenzimidazol tetrasulfonat | 0,50 | 1,00 | | | |
| Ethylhexyltriazon | | | | 4,00 | |
| Octocrylen | | 2,50 | 3,90 | | 2,50 |
| Diethyhexylbutamidotriazon | 1,00 | | | 2,00 | |
| Phenylbenzmidazolsulfonsäure | 0,50 | | | 3,00 | 2,00 |
| Methylen Bis-Benzotriazolyl | | | | 0,50 | |

(fortgesetzt)

| 3. W/O Sonnenschutz Emulsionen | | | | | |
|---|---|---|---|---|---|
| | **1** | **2** | **3** | **4** | **5** |
| Tetramethylbutylphenol | | | | | |
| Drometrizol Trisiloxan | | 1,00 | | | 1,50 |
| Terephthalidendicamphersulfonsäure | | | 1,00 | | 0,50 |
| Repellent 3535 | 8,00 | 10,00 | | 20,0 | 25 |
| "DEET" | | | 6,00 | | |
| Titandioxid T805 | | 2,00 | 1,50 | | 3,00 |
| Zinkoxid NDM | 1,00 | | | | 2,00 |
| Mineralöl | | | 10,0 | | 8,00 |
| C12-15 Alkylbenzoat | | | | 9,00 | |
| Dicaprylylether | 10,00 | | | | 7,00 |
| Butylenglycol Dicaprylat/Dicaprat | | | 2,00 | 8,00 | 4,00 |
| Dicaprylylcarbonat | 5,00 | | 6,00 | | |
| Dimethicon | | 4,00 | 1,00 | 5,00 | |
| Cyclomethicon | 2,00 | 25,00 | | | 2,00 |
| Shea Butter | | | 3,00 | | |
| Ethylhexylglycerin | | 0,30 | 1,00 | | 0,50 |
| Glycerin | 3,00 | 7,50 | | 7,50 | 2,50 |
| Glycine Soja | | 1,00 | 1,50 | | |
| $MgSO_4$ | 1,00 | 0,50 | | 0,50 | |
| $MgCl_2$ | | | 1,00 | | 0,70 |
| Vitamin E Acetat | 0,50 | | 0,25 | | 1,00 |
| DMDM Hydantoin | | 0,60 | | 0,20 | |
| Methylparaben | 0,50 | | | 0,15 | |
| Phenoxyethanol | 0,50 | 0,40 | | 1,00 | 0,60 |
| Ethanol | 3,00 | | 6,50 | | 1,00 |
| Parfüm | 0,20 | | | | 0,20 |
| Wasser | ad 100 | ad 100 | ad 100 | ad 100 | ad 100 |

| 4. Feststoffstabilisierte Emulsionen | | | | | |
|---|---|---|---|---|---|
| | **1** | **2** | **3** | **4** | **5** |
| Mineralöl | | | | | 16,0 |
| Octyldodecanol | 6,0 | | 7.5 | 7.5 | 5,0 |
| Caprylic/Capric Triglycerid | | | | | 6,0 |
| C12-15-Alkylbenzoat | 7,0 | 8,0 | 7,5 | 7,5 | |
| Butylen Glycol Dicaprylat/Dicaprat | 4,0 | 8,0 | | | |
| Dicaprylylether | | 8,0 | 7,5 | 7,5 | |

(fortgesetzt)

| 4. Feststoffstabilisierte Emulsionen | | | | | |
|---|---|---|---|---|---|
| | **1** | **2** | **3** | **4** | **5** |
| Dicaprylylcarbonat | 4,0 | | | | |
| Hydroxyoctacosanylhydroxystearat | 2,0 | 2,0 | 2,0 | 2,0 | 1,5 |
| PVP/Hexadecen Copolymer | | | | 1,0 | 0,7 |
| Disteardimoniumhectorit | 1,0 | 1,0 | 1,0 | 0,5 | 1,0 |
| Dimethicon | | 2,0 | | | |
| Cyclomethicon | | | | 2,0 | |
| Ethylhexylmethoxycinnamat | 5,0 | | 5,0 | | |
| Butylmethoxydibenzoylmethan | 3.00 | 2,0 | 0.50 | 1.80 | 1,0 |
| Ethylhexyltriazon | 2,0 | 2,0 | | | 1,0 |
| Methylen Bis-Benzotriazolyl Tetramethylbuthylphenol | | 3,00 | 4,00 | 2,50 | 1,00 |
| Drometrizol Trisiloxan | 2,00 | 1,50 | | | 4,00 |
| Terephthalidenicamphersulfonsäure | | 0,75 | | | |
| Bis-Ethylhexyloxyphenolmethoxyphenyltriazin | 2,5 | | 2,5 | | |
| Titandioxid + Alumina + Simethicon + Aqua | 1,5 | | 4,0 | 0,5 | 1,5 |
| Titandioxid + Trimethoxycaprylylsilan | | 4,0 | 2,0 | | |
| Zinkoxid | | | 2,0 | | |
| Phenylbenzimidazol Sulfonsäure | 2,0 | | | | |
| Dinatriumphenyldibenzimidazol tetrasulfonat | 2,50 | 1,00 | 0,60 | 1,50 | 3,00 |
| Bornitrid | | | | | 0,5 |
| Stärke/-Natriummetaphosphat-Polymer | 0,5 | | 1,5 | | |
| Korn Stärke Modifiziert | | 1,0 | | | |
| Acrylat Copolymer | | | | 0,25 | |
| Talk | | | | 2,0 | |
| Natriumchlorid | 1,0 | 1,0 | 1,0 | | |
| Repellent 3535 | 6,0 | 15 | 8,0 | 22,5 | 20 |
| Magnesiumsulfat | | | | | 0,70 |
| Natronlauge 45% | 0,5 | 0,5 | | | |
| Glycerin | 5,0 | 7,5 | 5,0 | 10,0 | 3,0 |
| Trinatrium EDTA | | 1,0 | 1,0 | | 1,0 |
| Propylencarbonat | 0,33 | 0,33 | 0,33 | | 0,33 |
| Methylparaben | 0,21 | 0,21 | 0,2 | 0,2 | 0,21 |
| Propylparaben | 0,07 | 0,07 | | | 0,07 |
| Phenoxyethanol | 0,5 | 0,5 | 0,5 | 0,5 | 0,5 |
| Hexamidindiisethionat | | | 0,08 | 0,08 | |
| Alkohol | | 5,0 | | | |
| Parfüm | q.s. | q.s. | q.s. | q.s. | q.s. |
| Wasser | ad 100 | ad 100 | ad 100 | ad 100 | ad100 |

| 5. Stifte | | | | |
|---|---|---|---|---|
| | 1 | 2 | 3 | 4 |
| Caprylic/Capric Triglycerid | 12 | 10 | 6 | |
| Octyldodecanol | 7 | 14 | 8 | 3 |
| Butylenglycol Dicaprylat/Dicaprat | | | | 12 |
| Pentaerythrityltetraisostearat | 10 | 6 | 8 | 7 |
| Polyglyceryl-3 Diisostearat | 2,5 | | | |
| Bis-Diglycerylpolyacyladipate-2 | 9 | 8 | 10 | 8 |
| Cetearylalkohol | 8 | 11 | 9 | 7 |
| Myristylmyristate | 3,5 | 3 | 4 | 3 |
| Beeswax | 5 | 5 | 6 | 6 |
| Cera Carnauba | 1,5 | 2 | 2 | 1,5 |
| Cera Alba | 0,5 | 0,5 | 0,5 | 0,5 |
| C16-40 Alkylstearat | | 1,5 | 1,5 | 1,5 |
| Dermacryl® 79 | 0,15 | | 2,5 | 3,0 |
| Repellent 3535 | 10,0 | 4,0 | 6,0 | 15,0 |
| Methylen Bis-Benzotriazolyl Tetramethylbuthylphenol | | | | 0,5 |
| Drometrizol Trisiloxan | 4,0 | 2,0 | 1,0 | 0,55 |
| Butyl Methoxydibenzoylmethan | | 1 | 1 | |
| Z-Cote® HP1 | | | | 4,5 |
| MT-100 TV | | 4 | 2,5 | |
| Titandioxid T 805 | | 3,6 | | 5 |
| Ethylhexylmethoxycinnamat | 3 | 3,6 | | 2,5 |
| Bis-Ethylhexyloxyphenolmethoxyphenyltriazin | 2,5 | | | 5 |
| Octocrylene | | | 7,5 | |
| Benzophenon-3 | | | 3,5 | |
| Ethylhexyltriazon | 2 | | | |
| Diethylhexylbutamidotriazon | | | | 3 |
| Tocopherylacetat | 0,5 | 1 | | |
| Ascorbylpalmitat | 0,05 | | 0,05 | |
| Buxus Chinensis | 2 | 1 | | 1 |
| Parfum, BHT | 0,1 | 0,25 | | 0,35 |
| Ricinus Communis | ad.100 | ad.100 | ad.100 | ad.100 |

| 6. PIT-Emulsionen | | | | | | |
|---|---|---|---|---|---|---|
| | 1 | 2 | 3 | 4 | 5 | 6 |
| Glycerinmonostearat SE | 0,50 | 2,00 | 3,00 | 5,00 | | |
| Glycerylisostearat | | | | | 3,50 | 4,00 |
| Isoceteth-20 | | 0,50 | | | 2,00 | |

(fortgesetzt)

| 6. PIT-Emulsionen | | | | | | |
|---|---|---|---|---|---|---|
| | **1** | **2** | **3** | **4** | **5** | **6** |
| Ceteareth-12 | | 5,00 | | 1,00 | | |
| Ceteareth-20 | | | | 2,00 | | 2,50 |
| PEG-100 Stearat | 5,00 | | 1,00 | | 1,00 | |
| Cetylalkohol | 2,50 | 1,00 | | 1,50 | | 0,50 |
| Cetylpalmitat | | | | 0,50 | | 1,00 |
| Cetyldimethicon Copolyol | 0,50 | | | | 0,50 | |
| Polyglyceryl-2 Dipolyhydroxystearat | | | | 0,75 | 0,25 | |
| Repellent® 3535 | 5,00 | 15,00 | 10,00 | 4,00 | 5,00 | 8,00 |
| Bis-Ethylhexyloxyphenolmethoxyphenyltriazin | | | 0,50 | 2,00 | | 3,00 |
| Butylmethoxydibenzoylmethan | 1,50 | | 1,00 | | 5,00 | 1,00 |
| Dinatriumphenyldibenzimidazol tetrasulfonat | | 2,00 | | | 1,00 | |
| Terephthalidendicamphersulfonsäure | | | 0,50 | | | |
| Methylen Bis-Benzotriazolyl Tetramethylbuthylphenol | | | 1,50 | | 4,00 | |
| Drometrizol Trisiloxan | 2,00 | 5,00 | | 1,00 | | 3,00 |
| Ethylhexylmethoxycinnamat | 8,00 | | | 4,50 | 5,00 | 8,00 |
| Dioctylbutamidotriazon | | | | 3,00 | 2,00 | 2,00 |
| Ethylhexyltriazon | | | 2,00 | 4,00 | | |
| Octocrylen | | | 5,00 | | | |
| Phenylbenzmidazolsulfonsäure | 1,00 | 5,00 | | 3,00 | | |
| C12-15 Alkyl Benzoat | 3,50 | | | | 6,50 | |
| Cocoglyceride | | 3,00 | | 3,00 | | |
| Dicaprylylether | 4,00 | | | | | |
| Butylenglycol Dicaprylat/Dicaprat | | 4,00 | | 3,00 | | |
| Dicaprylylcarbonat | | | | 0,50 | | |
| Phenyltrimethicon | 2,00 | | | 3,00 | | |
| Natriumcarbomer | | | 0,10 | | 0,30 | |
| PVP Hexadecen Copolymer | | | | 1,00 | 1,50 | |
| Glycerin | 10,0 | 5,00 | | 7,50 | | 10,0 |
| Fucogel®1000 | | | 2,50 | 6,00 | | |
| Tocopherol Acteate | 1,00 | | | 0,75 | 0,50 | |
| Shea Butter | | 2,00 | 3,50 | | | |
| Iodopropylbutylcarbamat | 0,12 | | | | 0,20 | |
| DMDM Hydantoin | | | | 0,10 | | |
| Methylparaben | | 0,50 | 0,25 | | 0,45 | |
| Phenoxyethanol | 0,50 | 0,40 | | 1,00 | | |
| Ethylhexyloxyglycerin | | 0,30 | | | 1,00 | 0,35 |

(fortgesetzt)

| 6. PIT-Emulsionen | | | | | | |
|---|---|---|---|---|---|---|
| | 1 | 2 | 3 | 4 | 5 | 6 |
| Ethanol | | | | 2,00 | | 6,00 |
| Trisodium EDTA | | 0,40 | | 0,15 | | 0,20 |
| Parfüm | 0,20 | | 0,20 | 0,20 | 0,45 | |
| Wasser | ad. 100 | ad. 100 | ad. 100 | ad. 100 | ad. 100 | ad. 100 |

**Patentansprüche**

**1.** Kosmetische und/oder dermatologische Zubereitungen, die mindestens einen UV-Filter mit dem Strukturelement des Benztriazols und mindestens eine insekten- und/oder spinnenabweisende Substanz (Repellent) enthalten.

**2.** Kosmetische und/oder dermatologische Zubereitungen nach Anspruch 1, **dadurch gekennzeichnet, dass** sie als Repellent 3-(N-n-Butyl-N-acetylamino)-propionsäureethylester enthalten.

**3.** Kosmetische und/oder dermatologische Zubereitungen nach einem der Ansprüche 1 oder 2, **dadurch gekennzeichnet, dass** sie als UV-Lichtschutzfilter 2,2'-Methylen-bis-(6-(2H-benzotriazol-2-yl)-4-(1,1,3,3-tetramethylbutyl)-phenol) [Tinosorb M (Ciba)] und/oder 2-(2H-benzotriazol-2-yl)-4-methyl-6-[2-methyl-3- [1,3,3,3 -tetramethyl-1-[(trimethylsilyl)oxy]disiloxanyl]propyl]-phenol [Mexoryl XL (Chimex)] enthalten.

**4.** Kosmetische und/oder dermatologische Zubereitungen nach einem der Ansprüche 1 bis 3, **dadurch gekennzeichnet, dass** sie Repellentien in einer Konzentration von 1 Gew. % bis 25 Gew. % und besonders bevorzugt in einer Konzentration von 4 Gew.% bis 25 Gew. %, jeweils bezogen auf das Gesamtgewicht der Zubereitung enthalten.

**5.** Kosmetische und/oder dermatologische Zubereitungen nach einem der Ansprüche 1 bis 4, **dadurch gekennzeichnet, dass** sie das oder die Benzotriazolderivate in einer Konzentration von 0,1% bis 15% und besonders bevorzugt in einer Konzentration von 0,5% bis 10%, jeweils bezogen auf das Gesamtgewicht der Zubereitung enthalten.

**6.** Kosmetische und/oder dermatologische Zubereitungen nach einem der Ansprüche 1 bis 5, **dadurch gekennzeichnet, dass** sie mindestens eine weitere UV-Filtersubstanz, gewählt aus der Gruppe Triazine, der bei Raumtemperatur flüssigen UV-Filter und/oder der anorganischen Pigmente enthalten.

**7.** Kosmetische und/oder dermatologische Zubereitungen nach einem der Ansprüche 1 bis 6, **dadurch gekennzeichnet, dass** sie mindestens eine weitere UV-A-Filtersubstanz und/oder einen Breitbandfilter, gewählt aus der Gruppe Dibenzoylmethanderivate [insbesondere das 4-(tert.-Butyl)-4'-methoxydibenzoylmethan], Phenylen-1,4-bis-(2-benzimidazyl)-3,3'-5,5'-tetrasulfonsäure-bis-natriumsalz, 1,4-di(2-oxo-10-Sulfo-3-bornylidenmethyl)-Benzol und dessen Salze und 2,4-Bis-{[4-(2-Ethylhexyloxy)-2-hydroxy]-phenyl}-6-(4-methoxyphenyl)-1,3,5-triazin, enthalten, wobei die weiteren Filtersubstanzen jeweils einzeln oder in beliebigen Kombinationen miteinander vorliegen können.

**8.** Kosmetische und/oder dermatologische Zubereitungen nach einem der Ansprüche 1 bis 7, **dadurch gekennzeichnet, dass** sie mindestens ein Flavonglycosid, insbesondere $\alpha$-Glucosylrutin, und/oder Vitamin E und/oder dessen Derivate enthalten.

**9.** Verwendung kosmetischer und/oder dermatologischer Zubereitungen nach einem der vorhergehenden Ansprüche als Sonnenschutzmittel und/oder Insekten und/oder Spinnen abwehrendes Mittel (Repellent).

**10.** Verwendung von Insektenrepellentien in kosmetischen und/oder dermatologischen Zubereitungen nach einem vorhergehenden Ansprüche zur Verbesserung der Verteilbarkeit von Benzotriazolderivaten auf der Haut.

**11.** Verwendung von Insektenrepellentien in kosmetischen und/oder dermatologischen Zubereitungen nach einem der vorangegangenen Ansprüche zur Erhöhung der Löslichkeit von Benzotriazolderivaten in kosmetischen und/oder dermatologischen Zubereitungen.

**12.** Verwendung von Insektenrepellentien in kosmetischen und/oder dermatologischen Zubereitungen nach einem der vorhergehenden Ansprüche zur Erhöhung des Lichtschutzfaktors benztriazolderivathaltiger kosmetischer und/oder dermatologischer Zubereitungen.